# EUROPEAN PATENT APPLICATION

(11) **EP 4 105 198 A1**
(43) Date of publication of application: **21.12.2022**
(21) Application number: 21752977.5
(22) Date of filing: 08.02.2021
(51) Int. Cl.: C07C 303/06, C07C 309/05

(54) **METHOD FOR PRODUCING ALKANEDISULFONIC ACID COMPOUND**

(30) Priority: 14.02.2020 JP 2020023220
(71) Applicant: Sumitomo Seika Chemicals Co., Ltd., Kako-gun, Hyogo 675-0145 (JP)
(72) Inventor: MASUHARA, Yusaku, Kako-gun, Hyogo 675-0145 (JP); ASHIBE, Seiya, Kako-gun, Hyogo 675-0145 (JP)
(74) Representative: Vossius & Partner Patentanwälte Rechtsanwälte mbB
(86) International application number: PCT/JP2021/004487
(87) International publication number: WO 2021/161942

(57) **Abstract**

Provided is an industrially advantageous method for producing an alkanedisulfonic acid compound easily and inexpensively with a high yield. The present invention is a method for producing an alkanedisulfonic acid compound, comprising reacting at least one alkanesulfonic acid compound having a specific structure and sulfur trioxide in the presence of at least one member selected from the group consisting of a sulfoxide compound and sulfone compound having a specific structure to thereby obtain an alkanedisulfonic acid compound.

## Description

### Technical Field

The present invention relates to a method for producing an alkanedisulfonic acid compound.

### Background Art

Conventionally, alkanedisulfonic acid compounds are known to be useful not only as drugs for treating leukemia in animals, but also as raw materials for functional materials such as stabilizers for secondary battery electrolytes, and are compounds with high utility value. Such alkanedisulfonic acid compounds are known to be produced by various methods.

For example, PTL 1 discloses a method for obtaining a methanedisulfonic acid compound by treating methane and sulfur trioxide with mercuric sulfate for 1.5 hours in an atmosphere of 5.8 MPa and 260°C. PTL 2 discloses a method for obtaining methanedisulfonic acid by reacting methanesulfonic acid and sulfur trioxide. PTL 3 discloses a method for obtaining methanedisulfonic acid by reacting dibromomethane and sulfite in the presence of tetrabutylammonium bromide and potassium iodide to obtain a methanesulfonic acid salt, and then using an ion exchange resin to obtain methanedisulfonic acid.

### Citation List

### Patent Literature

PTL 1: US2493038
PTL 2: US2842589
PTL 3: US2006/0155142

### Summary of Invention

### Technical Problem

However, the method disclosed in PTL 1 was not always suitable for industrial production because in addition to the need to use toxic mercury compounds, reaction conditions such as high temperature and high pressure were required. Moreover, the method disclosed in PTL 2 had problems that the yield of methanedisulfonic acid was low, which was disadvantageous in industrial production. Furthermore, the method disclosed in PTL 3 allowed reactions under atmospheric pressure; however, it was difficult to carry out on an industrial scale because of the complicated steps, the residual impurities of the catalyst, and the need for a large amount of ion exchange resin.

The present invention was made in view of the above problems, and its object is to provide an industrially advantageous method for producing an alkanedisulfonic acid compound easily and inexpensively with a high yield.

### Solution to Problem

The present inventors conducted extensive research to achieve the above object, and consequently found that the above object can be achieved by reacting a specific alkanesulfonic acid compound and sulfur trioxide in the presence of a specific sulfoxide compound and/or sulfone compound. Thus, the present invention has been completed.

Specifically, the present invention includes, for example, the main subjects described in the following items.

### Item 1.

A method for producing an alkanedisulfonic acid compound, comprising reacting at least one alkanesulfonic acid compound and sulfur trioxide in the presence of at least one member selected from the group consisting of a sulfoxide compound and a sulfone compound to thereby obtain an alkanedisulfonic acid compound;
the alkanesulfonic acid compound being represented by the following formula (1): 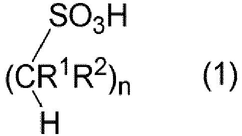 wherein in formula (1), R¹ and R² are the same or different, and each is a C₁₋₄ alkyl group optionally substituted with a halogen atom, or a hydrogen atom; and n is an integer of 1 to 4; when n is an integer of 2 to 4, n-number of R¹ may be the same or different, and n-number of R² may be the same or different;
the sulfoxide compound being represented by the following formula (2): 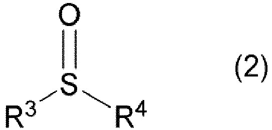 wherein in formula (2), R³ and R⁴ are the same or different, and each is a substituted or unsubstituted C₁₋₁₂ alkyl group, and R³ and R⁴ may bind to each other with or without a heteroatom together with the sulfur atom to which they bind to form a ring structure;
the sulfone compound being represented by the following formula (3): 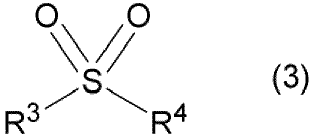 wherein in formula (3), R³ and R⁴ are the same or different, and each is a substituted or unsubstituted C₁₋₁₂ alkyl group, and R³ and R⁴ may bind to each other with or without a heteroatom together with the sulfur atom to which they bind to form a ring structure; and
the alkanedisulfonic acid compound being represented by the following formula (4): 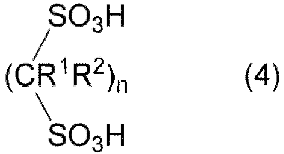 wherein in formula (4), R¹, R², and n are respectively the same as R¹, R², and n in formula (1) .

### Item 2.

The production method according to Item 1, wherein the sulfoxide compound is dimethylsulfoxide.

### Item 3.

The production method according to Item 1 or 2, wherein the sulfone compound is at least one member selected from the group consisting of sulfolane, 3-methylsulfolane, ethyl methyl sulfone, and ethyl isopropyl sulfone.

### Advantageous Effects of Invention

According to the production method of the present invention, it is possible to produce an alkanedisulfonic acid compound easily and inexpensively with a high yield, which is industrially advantageous.

### Description of Embodiments

Embodiments of the present invention are described in detail below. In the present specification, the term "comprising" includes "consisting essentially of" and "consisting of."

In the numerical range described in stages in the present specification, the upper or lower limit of the numerical range at one stage can be optionally combined with the upper or lower limit of the numerical range at another stage. In the numerical range described in the present specification, the upper or lower limit of the numerical range may be replaced with a value shown in the Examples or a value that can be uniquely derived from the Examples. Further, in the present specification, the numerical values connected by "to" mean the numerical range including the numerical values before and after "to" as the lower limit value and the upper limit value.

The method for producing an alkanedisulfonic acid compound of the present invention comprises reacting at least one alkanesulfonic acid compound and sulfur trioxide in the presence of at least one member selected from the group consisting of a sulfoxide compound and a sulfone compound to thereby obtain an alkanedisulfonic acid compound. This step is referred to below as "step A."

### Alkanesulfonic Acid Compound

The alkanesulfonic acid compound used in step A is represented by the following formula (1):

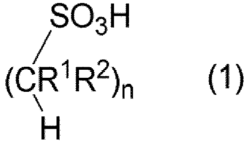

In formula (1), R¹ and R² are the same or different, and each is a C₁₋₄ alkyl group optionally substituted with a halogen atom, or a hydrogen atom; and n is an integer of 1 to 4. In formula (1), when n is an integer of 2 to 4, n-number of R¹ may be the same or different. Further, when n is an integer of 2 to 4, n-number of R² may be the same or different.

In R¹ and R² of formula (1), the type of C₁₋₄ alkyl group optionally substituted with a halogen atom is not particularly limited. Examples of the halogen atom include a fluorine atom, a chlorine atom, a bromine atom, and the like. Specific examples of the C₁₋₄ alkyl group optionally substituted with a halogen atom include a methyl group, an ethyl group, an n-propyl group, an iso-propyl group, an n-butyl group, an iso-butyl group, a sec-butyl group, a tert-butyl group, a fluoromethyl group, a trifluoromethyl group, a chloromethyl group, a chloroethyl group, a chloropropyl group, a bromomethyl group, and the like.

In formula (1), R¹ is preferably a hydrogen atom, a methyl group, an ethyl group, or an n-propyl group; and more preferably a hydrogen atom among these. R² is preferably a hydrogen atom, a methyl group, an ethyl group, or an n-propyl group; and more preferably a hydrogen atom among these. In formula (1), R¹ and R² are preferably the same.

In formula (1), when n is an integer of 2 to 4, that is, when n is 2, 3, or 4, n-number of R¹ in the compound represented by formula (1) may be the same or different. Similarly, n-number of R² may be the same or different. In formula (1), when n is an integer of 2 to 4, all of n-number of R¹ in the compound represented by formula (1) are preferably the same. When n is an integer of 2 to 4, all of n-number of R² in the compound represented by formula (1) are preferably the same.

In formula (1), n is particularly preferably 1. In this case, the yield of the object alkanedisulfonic acid compound tends to be particularly high.

The alkanesulfonic acid compound is not particularly limited, as long as it is a compound represented by formula (1). Preferred examples include methanesulfonic acid (in formula (1), R¹=R²=H, n=1), ethanesulfonic acid (in formula (1), R¹=CH₃, R²=H, n=1), 1-propanesulfonic acid (in formula (1), R¹=CH₂CH₃, R²=H, n=1), 2-propanesulfonic acid (in formula (1), R¹=R²=CH₃, n=1), 1-butanesulfonic acid (in formula (1), R¹=R²=H, n=4), and the like.

The alkanesulfonic acid compound used in step A can be obtained from a commercially available product, or can be obtained by a known method or a method easily conceived of from a known method. For example, with reference to Tetrahedron Letters (2009), 50(46), 6231-6232, the alkanesulfonic acid compound can be synthesized by a method of oxidizing a corresponding disulfide compound using methyltrioxorhenium(VII)/hydrogen peroxide as a catalyst. Alternatively, with reference to WO2004/058693, the alkanesulfonic acid compound can be synthesized by a method of oxidizing a corresponding thiol compound using hydrogen peroxide.

The alkanesulfonic acid compounds usable in step A can be used singly or in combination of two or more.

### Sulfoxide Compound and Sulfone Compound

In step A, either or both of a sulfoxide compound and a sulfone compound can be used.

The sulfoxide compound is represented by the following formula (2):

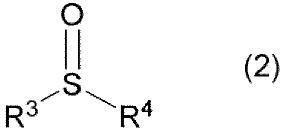

In formula (2), R³ and R⁴ are the same or different, and each is a substituted or unsubstituted C₁₋₁₂ alkyl group. In formula (2), R³ and R⁴ may bind to each other with or without a heteroatom together with the sulfur atom to which they bind to form a ring structure.

Further, the sulfone compound is represented by the following formula (3):

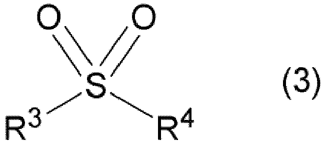

In formula (3), R³ and R⁴ are the same or different, and each is a substituted or unsubstituted C₁₋₁₂ alkyl group, and R³ and R⁴ may bind to each other with or without a heteroatom together with the sulfur atom to which they bind to form a ring structure. That is, R³ and R⁴ in formula (3) are respectively the same as R³ and R⁴ in formula (2) .

In R³ and R⁴ of formulas (2) and (3), when the C₁₋₁₂ alkyl group has a substituent, the type of substituent is not particularly limited. Specific examples of substituents include a halogen atom, an alkoxy group, a carboxyl group, a cyano group, a nitro group, a sulfo group, and the like.

In R³ and R⁴ of formulas (2) and (3), examples of the C₁₋₁₂ alkyl group include a methyl group, an ethyl group, an n-propyl group, an iso-propyl group, an n-butyl group, an iso-butyl group, a sec-butyl group, a tert-butyl group, an n-hexyl group, an n-octyl group, an n-decyl group, a cyclopropyl group, a cyclobutyl group, a cyclopentyl group, a cyclohexyl group, a cyclooctyl group, a norbornyl group, an adamantyl group, a cyclopentylmethyl group, a cyclohexylmethyl group, a 2-cyclohexylethyl group, and the like. These alkyl groups may have the substituents described above. Because the yield of the object alkanedisulfonic acid compound tends to be high, the number of carbon atoms is preferably 1 to 6. Therefore, among the above examples, the C₁₋₁₂ alkyl group is preferably a methyl group, an ethyl group, an n-propyl group, an iso-propyl group, an n-butyl group, an iso-butyl group, a sec-butyl group, a tert-butyl group, an n-hexyl group, a cyclopropyl group, a cyclobutyl group, a cyclopentyl group, a cyclohexyl group, or a cyclopentylmethyl group.

In formulas (2) and (3), R³ and R⁴ may bind to each other with or without a heteroatom together with the sulfur atom to which they bind to form a ring structure. That is, R³ and R⁴ may form a ring structure containing the sulfur atoms in the compounds represented by formulas (2) and (3) as ring members. In this case, for example, the carbon atoms in R³ and R⁴ can form chemical bonds to form a ring structure. For example, covalent bonds between the terminal carbon atoms in R³ and R⁴ can form a ring structure with the sulfur atoms. The ring structure is, for example, a saturated ring containing a sulfur atom. When the ring structure has a heteroatom in addition to the sulfur atom, the type of heteroatom is not particularly limited.

When the compound represented by formula (2) and the compound represented by formula (3) are both used in step A, R³ in formula (2) and R³ in formula (3) may be the same or different. Similarly, when the compound represented by formula (2) and the compound represented by formula (3) are both used in step A, R⁴ in formula (2) and R⁴ in formula (3) may be the same or different.

Specific examples of the sulfoxide compound represented by formula (2) include dimethylsulfoxide, ethylmethylsulfoxide, methyl n-propylsulfoxide, dipropylsulfoxide, dibutylsulfoxide, di-n-octylsulfoxide, didodecylsulfoxide, tetramethylenesulfoxide, and the like. Among these, the sulfoxide compound represented by formula (2) is preferably dimethylsulfoxide.

Specific examples of the sulfone compound represented by formula (3) include trimethylene sulfone, hexamethylene sulfone, trimethylene disulfone, tetramethylene disulfone, hexamethylene disulfone, sulfolane, 3-methylsulfolane, dimethyl sulfone, ethyl methyl sulfone, diethyl sulfone, methyl n-propyl sulfone, ethyl n-propyl sulfone, di-n-propyl sulfone, methyl isopropyl sulfone, ethyl isopropyl sulfone, diisopropyl sulfone, n-butyl methyl sulfone, n-butyl ethyl sulfone, t-butyl methyl sulfone, t-butyl ethyl sulfone, and the like. Among these, the sulfone compound represented by formula (3) is preferably sulfolane, 3-methylsulfolane, ethyl methyl sulfone, or ethyl isopropyl sulfone; more preferably sulfolane or 3-methylsulfolane; and even more preferably sulfolane.

When the compound represented by formula (2) and the compound represented by formula (3) are both used in step A, the sulfoxide compound and the sulfone compound are preferably at least one member selected from the group consisting of dimethylsulfoxide, sulfolane, 3-methylsulfolane, ethyl methyl sulfone, and ethyl isopropyl sulfone; more preferably at least one member selected from the group consisting of sulfolane, 3-methylsulfolane, ethyl methyl sulfone, and ethyl isopropyl sulfone; and particularly preferably contain at least sulfolane.

In step A, the sulfoxide compounds and the sulfone compounds can be used singly or in combination of two or more.

The sulfoxide compound and sulfone compound used in step A both can be obtained from commercially available products, or can be obtained by using known production methods or the like.

### Reaction in Step A

In step A, at least one alkanesulfonic acid compound and sulfur trioxide (SO₃) are reacted in the presence of at least one member selected from the group consisting of a sulfoxide compound and a sulfone compound.

The total amount of the sulfoxide compound and sulfone compound used in the reaction of step A is preferably 0.1 mol or more, more preferably 0.2 mol or more, and even more preferably 0.3 mol or more, per mol of sulfur trioxide, because the yield of the object alkanedisulfonic acid compound tends to be high. Further, the total amount of the sulfoxide compound and sulfone compound used in the reaction of step A is preferably 10 mol or less, more preferably 8 mol or less, and even more preferably 6 mol or less, per mol of sulfur trioxide, in terms of being more economical.

The amount of sulfur trioxide used in the reaction of step A is preferably 0.1 mol or more, more preferably 0.2 mol or more, and even more preferably 0.3 mol or more, per mol of the alkanesulfonic acid compound, because the yield of the object alkanedisulfonic acid compound tends to be high. Further, the amount of sulfur trioxide used in the reaction of step A is preferably 10 mol or less, more preferably 8 mol or less, and even more preferably 6 mol or less, per mol of the alkanesulfonic acid compound, in terms of being more economical. The phrase "per mol of the alkanesulfonic acid compound" as used herein means that when two or more alkanesulfonic acid compounds are used, the total amount thereof is 1 mol.

In the reaction of step A, for example, raw material A comprising the alkanesulfonic acid compound, sulfur trioxide, and at least one member selected from the group consisting of the sulfoxide compound and the sulfone compound is placed in a reaction vessel, and the reaction can be carried out in the reaction vessel. In this case, raw material A can be dissolved or dispersed in a solvent, if necessary. When a solvent is used, the amount thereof is not particularly limited. For example, the amount of solvent can be set to 1500 parts by mass or less, and preferably 1000 parts by mass or less, based on 100 parts by mass of the total amount of the alkanesulfonic acid compound.

When raw material A is dissolved or dispersed in a solvent, usable solvents can be suitably selected within the range in which the effect of the present invention is not impaired. Examples of such solvents include hydrocarbon-based solvents, halogen-based solvents, ether-based solvents, ketone-based solvents, ester-based solvents, amide-based solvents, nitrile-based solvents, sulfuric acid, and the like. Examples of hydrocarbon-based solvents include toluene, xylene, monochlorobenzene, dichlorobenzene, trichlorobenzene, hexane, heptane, decane, and the like. Examples of halogen-based solvents include dichloromethane, 1,2-dichloroethane, and the like. Examples of ether-based solvents include diethyl ether, ethylene glycol dimethyl ether, diisopropyl ether, diphenyl ether, tetrahydrofuran, dioxane, methyl-tert-butyl ether, cyclopentyl methyl ether, and the like. Examples of ketone-based solvents include acetone, methyl ethyl ketone, methyl isobutyl ketone, and the like. Examples of ester-based solvents include ethyl acetate, butyl acetate, and the like. Examples of amide-based solvents include dimethylformamide, dimethylacetamide, N-methylpyrrolidone, and the like. Examples of nitrile-based solvents include acetonitrile and the like.

Raw material A used in step A may contain other additives and the like, in addition to the solvent. Alternatively, raw material A may consist of the alkanesulfonic acid compound, sulfur trioxide (SO₃), and at least one member selected from the group consisting of a sulfoxide compound and a sulfone compound.

The type of reaction vessel used in the reaction of step A is not particularly limited. For example, a wide range of known reaction vessels can be used.

The conditions of the reaction performed in step A are also not particularly limited. For example, the temperature of the reaction performed in step A is not particularly limited, and can be suitably set depending on the type and amount of raw material used and other conditions. For example, the reaction temperature can be set to 0 to 200°C, and because the yield of the object tends to be higher, preferably 50 to 180°C, more preferably 80 to 170°C, and even mover preferably 100 to 160°C. The reaction time can be suitably set depending on the reaction temperature, and can be set to about 1 to 48 hours, for example.

In the reaction of step A, the method of adjusting the reaction temperature is also not particularly limited. For example, the reaction vessel containing raw material A can be heated to set an appropriate reaction temperature.

The reaction can be carried out while stirring raw material A with a stirrer or the like. Further, the reaction can be carried out by placing all the raw materials into the reaction vessel in no particular order, and then maintaining the temperature at a predetermined temperature. Alternatively, the reaction may be carried out by dropping any raw material or multiple raw materials.

The reaction of step A can be carried out under pressurized, depressurized, or atmospheric conditions. Further, the reaction of step A can also be carried out under an inert gas atmosphere, such as nitrogen or argon. The reaction can also be carried out while blowing an inert gas.

As a result of the reaction of step A, the object alkanedisulfonic acid compound is produced. The alkanedisulfonic acid compound is represented by the following formula (4):

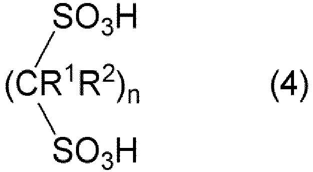

In formula (4), R¹, R², and n are respectively the same as R¹, R², and n in formula (1) .

Specific examples of the alkanedisulfonic acid compound represented by formula (4) include methanedisulfonic acid (R¹=R²=H, n=1), ethanedisulfonic acid (R¹=CH₃, R²=H, n=1), 1-propanedisulfonic acid (R¹=3H₂CH₃, R²=H, n=1), 2-propanedisulfonic acid (R¹=R²=CH₃, n=1), 1-butanedisulfonic acid (R¹=R²=H, n=4), and the like.

The alkanedisulfonic acid compound obtained in step A can be isolated by conventionally known purification and isolation operations, and the method thereof is not particularly limited. For example, the reaction liquid obtained after the reaction of step A can be extracted with a solvent or the like and washed, followed by crystallization, thereby isolating the alkanedisulfonic acid compound obtained in step A. When the obtained alkanedisulfonic acid compound is used as a raw material for other reactions, the compound is not necessarily isolated. The reaction liquid containing the alkanedisulfonic acid compound can be directly used in the subsequent reaction. For example, the alkanedisulfonic acid compound obtained in step A can be used as a raw material for producing a methylene disulfonate compound.

In general, in the production of an alkanedisulfonic acid compound using sulfur trioxide, the reaction between sulfur trioxide and an alkanesulfonic acid compound is less likely to occur, and thus the yield of the alkanedisulfonic acid compound is low. In contrast, as in step A, when the reaction is carried out in the presence of at least one member selected from the group consisting of a sulfoxide compound and a sulfone compound, these compounds have the effect of effectively reacting sulfur trioxide with an alkanesulfonic acid compound. Therefore, the reaction between sulfur trioxide and the alkanesulfonic acid compound is likely to occur, which improves the yield of the alkanedisulfonic acid compound to be produced.

The present disclosure includes all of any combinations of the configuration requirements described in the present specification. Further, the various characteristics (properties, structures, functions, etc.) described for each embodiment of the present disclosure described above may be combined in any way in identifying the main subjects included in the present disclosure. That is, the present disclosure includes all the main subjects comprising various combinations of the characteristics that can be combined described in the present specification.

### Examples

The present invention is described in more detail below with reference to Examples; however, the present invention is not limited to these Examples.

### Example 1

In a four-necked flask equipped with a stirrer, a cooling tube, a thermometer, and a dropping funnel, 19.2 g (0.20 mol) of methanesulfonic acid, 20.0 g (0.17 mol) of sulfolane, and 16.0 g (0.20 mol) of sulfur trioxide were placed as raw material A. While stirring raw material A in the flask, the temperature was raised to 150°C, and the reaction was continued at this temperature for 5 hours to thereby obtain a reaction liquid. An appropriate amount of the obtained reaction liquid was taken for ion chromatography analysis. Based on the area values of the peaks obtained in this analysis, the production yield of the product, i.e., methanedisulfonic acid represented by formula (4) (compound wherein R¹ and R² are hydrogen atoms, and n is 1), was calculated using the methanesulfonic acid placed as a reference. As a result, the production yield of the resulting methanedisulfonic acid was 74 mol%.

### Example 2

In the preparation of raw material A, methanedisulfonic acid represented by formula (4) (compound wherein R¹ and R² are hydrogen atoms, and n is 1) was produced in the same manner as in Example 1, except that the amount of sulfolane used was changed to 80.0 g (0.67 mol). Then, the production yield was calculated. As a result, the production yield of the resulting methanedisulfonic acid was 77 mol%.

### Example 3

In the preparation of raw material A, methanedisulfonic acid represented by formula (4) (compound wherein R¹ and R² are hydrogen atoms, and n is 1) was produced in the same manner as in Example 1, except that the amount of sulfolane used was changed to 80.0 g (0.67 mol), and the amount of sulfur trioxide used was changed to 32.0 g (0.40 mol). Then, the production yield was calculated. As a result, the production yield of the resulting methanedisulfonic acid was 83 mol%.

### Comparative Example 1

In the preparation of raw material A, methanedisulfonic acid represented by formula (4) (compound wherein R¹ and R² are hydrogen atoms, and n is 1) was produced in the same manner as in Example 1, except that sulfolane was not used. Then, the production yield was calculated. As a result, the production yield of the resulting methanedisulfonic acid was 40 mol%.

The above results revealed that according to the production methods performed in the Examples, the presence of step A allowed the reaction to proceed well, and the object methanedisulfonic acid represented by formula (4) could be obtained with a high production yield. That is, it was demonstrated that according to the production method of the present invention, it is possible to produce alkanedisulfonic acid compounds easily and inexpensively with a high yield, which is industrially advantageous.

## Claims

1. A method for producing an alkanedisulfonic acid compound, comprising reacting at least one alkanesulfonic acid compound and sulfur trioxide in the presence of at least one member selected from the group consisting of a sulfoxide compound and a sulfone compound to thereby obtain an alkanedisulfonic acid compound;
the alkanesulfonic acid compound being represented by the following formula (1): 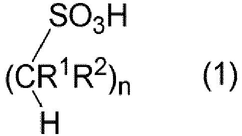 wherein in formula (1), R¹ and R² are the same or different, and each is a C₁₋₄ alkyl group optionally substituted with a halogen atom, or a hydrogen atom; and n is an integer of 1 to 4; when n is an integer of 2 to 4, n-number of R¹ may be the same or different, and n-number of R² may be the same or different;
the sulfoxide compound being represented by the following formula (2): 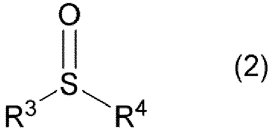 wherein in formula (2), R³ and R⁴ are the same or different, and each is a substituted or unsubstituted C₁₋₁₂ alkyl group, and R³ and R⁴ may bind to each other with or without a heteroatom together with the sulfur atom to which they bind to form a ring structure;
the sulfone compound being represented by the following formula (3): 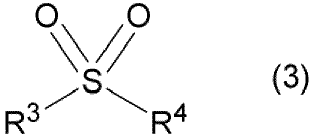 wherein in formula (3), R³ and R⁴ are the same or different, and each is a substituted or unsubstituted C₁₋₁₂ alkyl group, and R³ and R⁴ may bind to each other with or without a heteroatom together with the sulfur atom to which they bind to form a ring structure; and
the alkanedisulfonic acid compound being represented by the following formula (4): 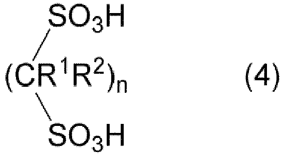 wherein in formula (4), R¹, R², and n are respectively the same as R¹, R², and n in formula (1).

2. The production method according to claim 1, wherein the sulfoxide compound is dimethylsulfoxide.

3. The production method according to claim 1 or 2,
wherein the sulfone compound is at least one member selected from the group consisting of sulfolane, 3-methylsulfolane, ethyl methyl sulfone, and ethyl isopropyl sulfone.
